# FASCICULE DE BREVET EUROPEEN

(11) **EP 1 075 223 B1**
(45) Date de publication et mention de la délivrance du brevet: **16.11.2005**
(21) Numéro de dépôt: 99916955.0
(22) Date de dépôt: 29.04.1999
(51) Int. Cl.: A61B 17/70

(54) **SYSTEME D'OSTEOSYNTHESE RACHIDIENNE POUR UNE FIXATION ANTERIEURE**
WIRBELSÄULEN-OSTEOSYNTHESESYSTEM INSBESONDERE ZUR VORDEREN FIXIERUNG
BACKBONE OSTEOSYNTHESIS SYSTEM FOR ANTERIOR FIXING

(30) Priorité: 29.04.1998 FR 9805387; 09.10.1998 FR 9812662
(43) Date de publication de la demande: 14.02.2001
(73) Titulaire: Stryker Spine, 33610 Cestas (FR)
(72) Inventeur: ASSAKER, Richard, B-7540 Kain (BE); CONCHY, Frédéric, F-33650 Saint-Médard-d'Eyrans (FR); LE COUEDIC, Régis, F-33610 Cestas (FR)
(74) Mandataire: Warcoin, Jacques
(86) Numéro de dépôt international: PCT/FR1999/001019
(87) Numéro de publication internationale: WO 1999/055246

(56) Documents cités:
- WO-A-96/27340
- DE-A- 4 433 360
- DE-U- 9 412 744
- US-A- 5 613 968

## Description

L'invention concerne les systèmes d'ostéosynthèse rachidienne notamment pour fixation antérieure.

On connaît des systèmes d'ostéosynthèse rachidienne pour fixation antérieure dans lesquels les éléments de liaison sont constitués par des plaques et d'autres dans lesquels ils sont constitués par des tiges. Du fait de leur encombrement, les systèmes à base de plaques sont difficilement, voire pas du tout utilisables par voie endoscopique. De plus, leur taille limitée (longueur) ne permet de les utiliser que pour de simples corporectomies concernant une seule vertèbre, voire deux. Il est impossible de traiter une scoliose avec ce genre d'implant. Enfin, les plaques sont difficilement adaptables à la morphologie de la vertèbre où elles sont ancrées. Par ailleurs, les systèmes à base de tiges comprennent généralement des connecteurs assez volumineux qui ne sont pas toujours utilisables par voie endoscopique.

On connaît par ailleurs d'après le document FR-2 731 344, qui a pour homologue le WO-96/27340, un dispositif d'ostéosynthèse rachidienne comprenant un connecteur à deux branches pouvant serrer entre elles une tige de liaison, les branches étant aptes à être enfilées sur une vis pédiculaire vertébrale. Toutefois, ce connecteur, bien adapté pour une fixation en partie postérieure du rachis sur les pédicules vertébraux, n'assure pas une stabilité suffisante en vue d'une fixation en partie antérieure du rachis.

Le document DE-4 433 360 divulgue un système d'ostéosynthèse selon le préambule de la revendication 1.

Un but de l'invention est de fournir un système d'ostéosynthèse rachidienne d'un type différent, adapté pour une fixation antérieure, facile à monter, assurant une bonne stabilité du système sur le rachis et compatible avec un montage par voie endoscopique.

En vue de la réalisation de ce but, on prévoit selon l'invention un système d'ostéosynthèse rachidienne conforme à la revendication 1.

Ainsi, le connecteur peut être rendu peu volumineux afin d'être mis en place par voie normale ou endoscopique. De plus, la fixation du connecteur à la vertèbre au moyen des deux vis permet d'assurer un positionnement précis, stable et sûr du connecteur et donc de l'élément de liaison. Ce connecteur est facile à assembler à l'élément de liaison et aux vis. Ces avantages rendent ce connecteur particulièrement adapté à une fixation en partie antérieure du rachis. Le connecteur sera préférablement monobloc.

Avantageusement, le prolongement présente un évidement de réception de la deuxième vis.

Avantageusement, le prolongement présente une empreinte sphérique à un bord de l'évidement destiné à être opposé à la vertèbre.

Ainsi, on peut régler l'angle de la deuxième vis par rapport au connecteur pour mieux adapter le système à là conformation de la vertèbre.

Avantageusement, une des branches destinée à être opposée à la vertèbre présente un évidement de réception de la première vis, et une empreinte sphérique à un bord de l'évidement destiné à être opposé à la vertèbre.

Ainsi, on peut régler l'angle de la première vis par rapport au connecteur pour mieux adapter le système à la conformation de la vertèbre.

Avantageusement, la branche prolongée est apte à être cintrée manuellement, notamment au moyen d'un outil.

Ainsi, on peut adapter la forme du connecteur à celle de la vertèbre et notamment positionner le connecteur très près de celle-ci.

Avantageusement, la première vis comporte une tête et une collerette distincte de la tête et apte à coopérer avec une des branches destinée à être adjacente à la vertèbre, pour immobiliser le connecteur en rotation par rapport à la première vis.

Ainsi, lors du montage, on obtient une immobilisation préalable du connecteur par rapport à la première vis, facilitant la mise en place des autres éléments et permettant d'effectuer des corrections de positions par réglage, le tout avant serrage final du système installé.

Avantageusement, la collerette présente une face, notamment conique, apte à immobiliser le connecteur par friction.

Avantageusement, la première vis présente un orifice fileté, le système comprenant une vis de serrage apte à constituer une liaison vis-écrou avec cet orifice et à prendre appui sur une des branches destinée à être opposée à la vertèbre pour serrer les branches.

Ainsi, on peut d'abord mettre en place la première vis, le connecteur et la deuxième vis, puis seulement ensuite installer la vis de serrage pour procéder au serrage de l'ensemble.

Avantageusement, le système comprend une bague apte à être enfilée sur la tige et reçue encre les branches, le connecteur et la bague étant conformés pour permettre un réglage de l'orientation de la tige dans deux plans perpendiculaires entre eux, avant le serrage des branches.

Avantageusement, les deux branches font partie d'une même pièce déformable élastiquement pour rapprocher les branches l'une de l'autre.

Avantageusement, le connecteur est apte à être fixé à la vis vertébrale et au premier élément de liaison en choisissant une position angulaire de l'élément de liaison par rapport au connecteur.

On a également remarqué que le dispositif du document FR-2 731 344 précité ne fournit pas toujours une rigidité suffisante en vue d'une fixation en partie antérieure du rachis.

Par conséquent, un but de l'invention est subsidiairement de fournir un système adapté pour une fixation antérieure en assurant une rigidité particulièrement élevée.

En vue de la réalisation de ce but, on prévoit avantageusement que le système comprend un deuxième élément de liaison allongé, le connecteur étant apte à être fixé simultanément aux deux éléments de liaison.

Ainsi, la présence des deux éléments de liaison confère au système une très grande rigidité, sans compliquer son assemblage, sans accroître le volume de ses différentes pièces (ce qui le rend compatible avec un montage par voie endoscopique), et tout en conservant la possibilité de réglage de la position angulaire du connecteur par rapport au premier élément de liaison. Le système selon l'invention ne requiert pas un cintrage identique sur les deux éléments de liaison. De plus, le nombre de connecteurs peut rester peu élevé.

Avantageusement, le système est agencé de sorte que le deuxième élément de liaison ne peut être fixé au connecteur que dans une seule position angulaire par rapport au connecteur.

Ainsi, la forme du deuxième élément de liaison commande la position angulaire relative des connecteurs fixés à celui-ci. On peut donc prévoir et choisir à l'avance cette position angulaire suivant la courbure préalable que l'on donne à cet élément de liaison, à la fabrication ou mieux lors de l'intervention chirurgicale.

Avantageusement, le deuxième élément de liaison présente une résistance au cintrage plus faible que le premier élément de liaison.

Ainsi, le premier élément de liaison a principalement une fonction de support des connecteurs et le deuxième élément de liaison a principalement une fonction de positionnement angulaire relatif des connecteurs.

Avantageusement, les branches sont aptes à serrer simultanément les deux éléments de liaison.

Avantageusement, le système est agencé de sorte que le deuxième élément de liaison, lorsqu'il est fixé au connecteur, s'étend dans une trajectoire de la deuxième vis vertébrale pour son désengagement du connecteur.

Ainsi, on prévient tout début de sortie intempestive de la deuxième vis.

Avantageusement, le système comprend un deuxième connecteur, les deux connecteurs étant aptes à être fixés simultanément chacun aux deux éléments de liaison.

Avantageusement, le système est destiné à être fixé en partie antérieure du rachis.

D'autres caractéristiques et avantages de l'invention apparaîtront encore dans la description suivante de deux modes préférés de réalisation donnés à titre d'exemples non limitatifs. Aux dessins annexés :
- la figure 1 est une vue en perspective du système selon un premier mode de réalisation de l'invention ;
- la figure 2 est une vue partielle en perspective éclatée du système de la figure 1 ;
- les figures 3 et 4 sont deux vues en perspective, respectivement de dessus et de dessous, montrant un des connecteurs du système de la figure 1 ;
- la figure 5 est une vue, moitié en élévation et moitié en coupe axiale, d'une bague du système de la figure 1 ;
- la figure 6 est une vue, pour partie de dessus et pour partie en coupe, du connecteur de la figure 3 recevant la tige ;
- la figure 7 est une vue partielle en perspective montrant la tête de la vis principale ;
- la figure 8 montre le système de la figure 1 fixé sur des vertèbres ;
- la figure 9 est une vue en perspective du système selon un deuxième mode de réalisation de l'invention ;
- la figure 10 est une vue partielle en perspective éclatée du système de la figure 9 ;
- les figures 11 et 12 sont deux vues en perspective, respectivement de dessus et de dessous, montrant un des connecteurs du système de la figure 9 ; et .
- la figure 13 montre le système de la figure 9 fixé sur des vertèbres.

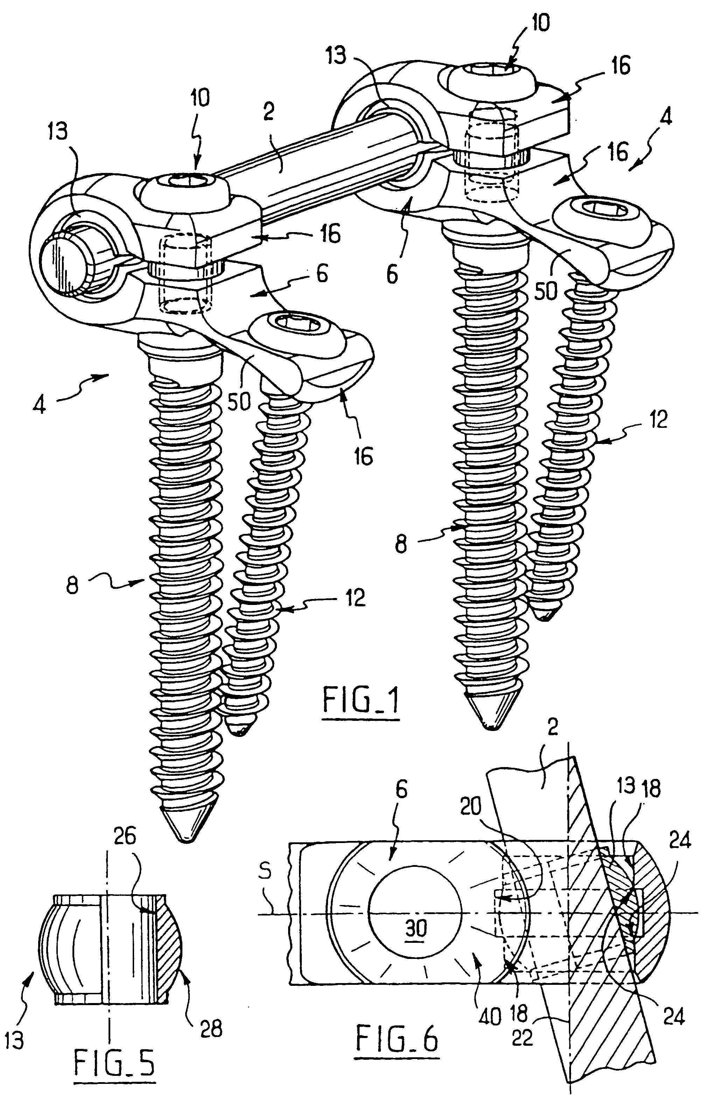

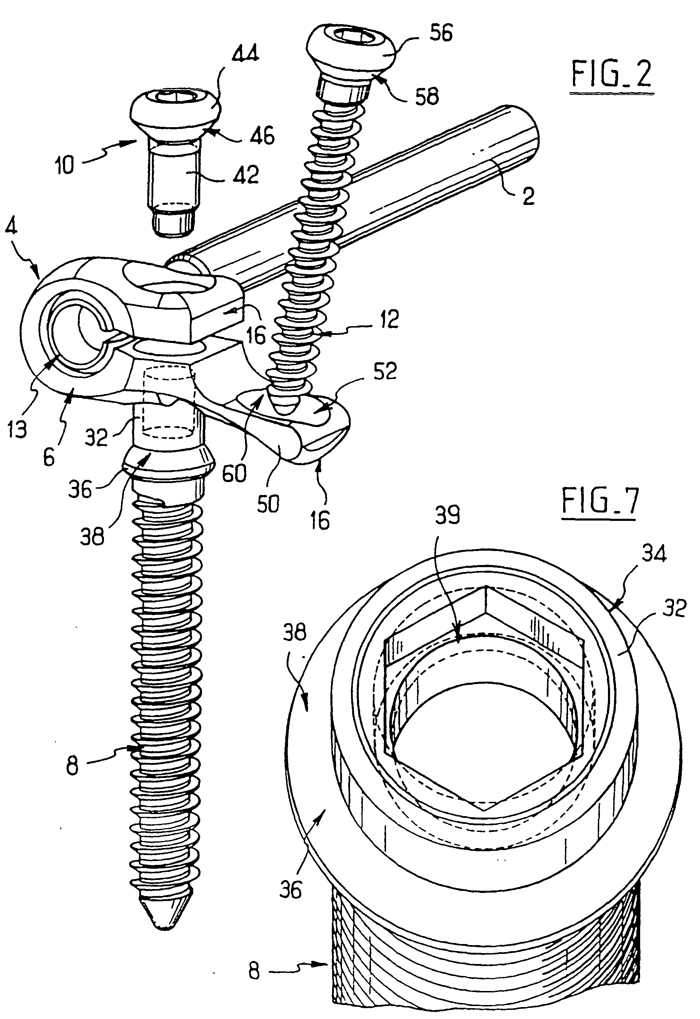

En référence aux figures 1 à 8, le système selon l'invention comprend dans le premier mode de réalisation une tige de liaison allongée 2 à section circulaire et plusieurs sous-ensembles 4 à connecteur aptes à être fixés à celle-ci. Chacun de ces sous-ensembles, dont deux sont visibles sur la figure 1 et un seul est visible sur la figure 2, comprend un connecteur 6, une première vis vertébrale ou vis principale 8, une vis de serrage 10, une deuxième vis vertébrale ou vis secondaire 12, et une bague 13.

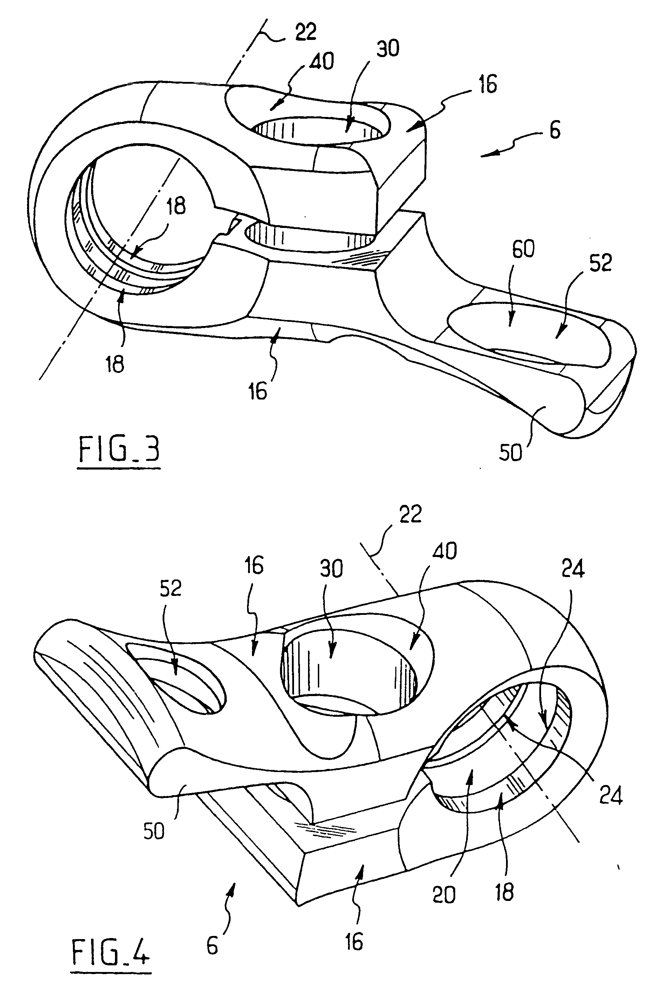

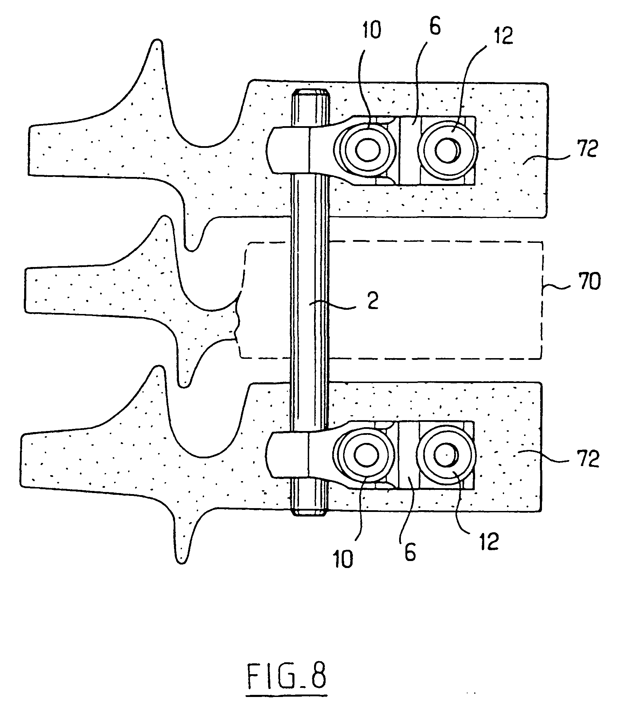

En référence aux figures 3 et 4, le connecteur 6 comporte deux branches 16 s'étendant en regard et à distance l'une de l'autre en donnant au connecteur un profil général en "U". Le connecteur 6 comporte un plan de symétrie S perpendiculaire à la largeur des branches 16 et parallèle à leur longueur. En référence à la figure 6, au niveau de la naissance des branches 16, le connecteur présente deux faces internes cylindriques coaxiales 18, 20 d'axe 22 perpendiculaire au plan « S » et de rayons différents, la face 20 de plus grand rayon est en deux parties disjointes et s'étend de part et d'autre de la face 18 de plus petit rayon, laquelle est traversée par le plan « S ». Les deux faces 18, 20 forment à leurs jonctions deux arêtes circulaires 24 d'axe 22.

La bague 13 présente une face interne cylindrique 26 et une face externe sphérique 28 coaxiales. La face interne cylindrique 26 a un rayon environ égal à celui de la tige 2 de sorte que la bague 13, fendue sur un côté suivant son axe, peut être reçue par ajustement glissant sur la tige. Par ailleurs, la bague 13 peut être logée entre les branches 16 en regard des faces cylindriques 18, 20. La face externe sphérique 28 de la bague a un rayon adapté pour que dans cette position, les arêtes 24 du connecteur 6 soient en contact linéaire avec la face externe sphérique 28 de la bague 13 et lui servent d'appuis. Dans cette position, avant serrage des branches 16, la position angulaire de la tige 2 enfilée dans la bague 13 peut être réglée dans deux plans perpendiculaires entre eux sur une amplitude par exemple de 15° de part et d'autre d'une position moyenne de la tige dans laquelle la tige est perpendiculaire au plan « S ».

Les branches 16 présentent deux évidements cylindriques lisses respectifs qui sont en l'espèce des orifices traversants 30 s'étendant coaxialement en regard l'un de l'autre. La vis principale 8 est une vis vertébrale bicorticale et présente un corps fileté à cette fin de façon connue en soi. Elle présente une tête 32 ayant une face externe cylindrique lisse 34. A la jonction entre la tête et le corps, la vis comporte une collerette annulaire 36 ayant une face inférieure plane perpendiculaire à un axe longitudinal de la vis et une face supérieure tronconique 38 avec la section la plus étroite du tronc de cône situé du côté de la tête 32 de la vis. La tête 32 présente un orifice fileté 39 coaxial au corps de la vis et, ménagée dans la face filetée de l'orifice 39, une forme non circulaire telle qu'une empreinte hexagonale à six pans. La vis de serrage 10 comporte un corps fileté 42 apte à former une liaison vis-écrou avec cet orifice 39, et une tête de vis 44 dans laquelle est formée une empreinte hexagonale à six pans. La tête 44 a une face externe inférieure sphérique convexe 46 dont la section la plus étroite est située vers la pointe de la vis.

L'une des branches 16, que pour la clarté nous appellerons ici branche inférieure, présente un prolongement 50 s'étendant en direction opposée aux faces cylindriques 18, 20 du connecteur. Il s'agit ici de la branche destinée à être adjacente à la vertèbre. Les deux branches 16 sont aptes à être enfilées simultanément sur la tête 32 de la vis principale 8 introduite à partir de la branche inférieure contre laquelle la face supérieure 38 de la collerette 36 vient en butée. On introduit ensuite la vis de serrage 10 dans la tête 32 de la vis principale 8 à partir de la branche supérieure 16. Le serrage de la vis 10 dans la tête 32 de la vis principale 8 provoque le rapprochement des branches 16 l'une de l'autre et le blocage par friction de la tige 2 dans la position choisie par rapport au connecteur 6.

L'orifice 30 de la branche inférieure 16 a un bord inférieur, opposé à la branche supérieure et destiné à être du côté de la vertèbre, présentant une empreinte sphérique concave 40 destinée à venir en contact avec la face supérieure 38 de la collerette 36 pour réaliser par friction un blocage à rotation du connecteur 6 par rapport à l'axe de la vis principale 8. L'orifice 30 de la branche supérieure 16 a un bord supérieur, opposé à la branche inférieure et destiné à être opposé à la vertèbre, présentant une empreinte sphérique concave 40 destinée à venir en contact avec la face inférieure sphérique convexe 46 de la tête 44 de la vis de serrage 10 et permettant de fixer celle-ci ainsi que la vis principale 8 en réglant l'orientation angulaire de la vis principale 8 par rapport au connecteur.

Le prolongement 50 présente un évidement sous la forme d'un orifice traversant 52. La branche inférieure 16 est courbée au niveau du prolongement 50 dans une direction opposée à la branche supérieure 16 de sorte que les axes des orifices 30 et 52 qu'elle porte ne sont pas tout à fait parallèles. La vis secondaire 12 est une vis vertébrale, ici monocorticale, présentant un corps fileté et une tête 56 à face inférieure sphérique convexe 58 dont la section la plus étroite se trouve du côté du corps. Sa tête présente une empreinte à six pans. L'orifice 52 du prolongement a un bord supérieur, orienté du côté de l'autre branche 16 et destiné à être opposé à la vertèbre, présentant une empreinte sphérique concave 60 destinée à venir en contact avec la face inférieure sphérique convexe 58 de la tête 56 de la vis secondaire 12 en permettant de régler l'orientation angulaire de cette vis par rapport au connecteur 6.

On retrouvera dans les documents FR-2 731 344 et WO-96/27340 homologues précités certaines des caractéristiques du connecteur 6 qui n'ont pas été développées ici en grand détail.

La branche inférieure 16 peut être cintrée pour accentuer ou réduire sa courbure en vue de mieux s'adapter à la forme de la partie antérieure de sa vertèbre de destination. Une fois cintrée, cette branche 16 n'est plus sollicitée en flexion puisqu'elle est fixée à la vertèbre par deux vis 8, 12 sur sa longueur. Les deux vis principale 8 et secondaire 12 sont autotaraudeuses et comportent des filetages à os.

Dans une variante de réalisation, la vis principale 8 ne présente pas une empreinte à six pans dans son orifice fileté 39, mais la collerette 36 a une forme hexagonale ou présente deux méplats parallèles et diamétralement opposés l'un à l'autre pouvant coopérer avec une clé de serrage pour mettre cette vis 8 en rotation par rapport au connecteur 6.

Dans le présent exemple, le connecteur 6 est d'une seule pièce. Les différentes parties du système sont en métal biocompatible.

La mise en place d'un tel dispositif s'effectue de la manière suivante en référence à la figure 8 : après exposition de la vertèbre affectée 70 et des deux vertèbres adjacentes 72, on réalise une corporectomie tout en préservant, quand cela est possible, les plateaux respectifs de celles-ci. Pour chaque sous-ensemble, on réalise un avant-trou sur le côté latéral de la vertèbre 72 associée à égale distance des plateaux supérieur et inférieur, ainsi qu'à la limite du quart le plus postérieur du corps vertébral. On insère ensuite la vis principale 8 dans cet avant-trou jusqu'à la collerette d'arrêt 36. le connecteur 6 est ensuite positionné sur ladite vis principale 8, bloqué en translation par la portée conique 38 de ladite vis principale 8 venant s'adapter à l'empreinte 40 du connecteur 6. On repère l'adaptation du connecteur 6 sur la vertèbre, que l'on peut ajuster par le retrait dudit connecteur pour cintrer la branche inférieure 16 qui est sa partie la plus antérieure.

On vient ensuite visser la vis secondaire 12 par rapport à la vis principale 8 dans le second orifice 52 de la branche inférieure 16 jusqu'au contact du logement sphérique 60 du prolongement prévu à cet effet avec la partie sphérique 58 de ladite vis secondaire 12. Il est souhaitable de positionner le connecteur 6 le plus parallèle possible aux plateaux.

Après avoir instrumenté les deux vertèbres adjacentes 72, on positionne la tige 2 dans les bagues des connecteurs 6 et on règle sa position angulaire sur chaque sous-ensemble. Le serrage final s'opère grâce à la vis de serrage 10 qui s'insère dans la vis principale 8 et comprime ainsi le connecteur 6 pour serrer la tige.

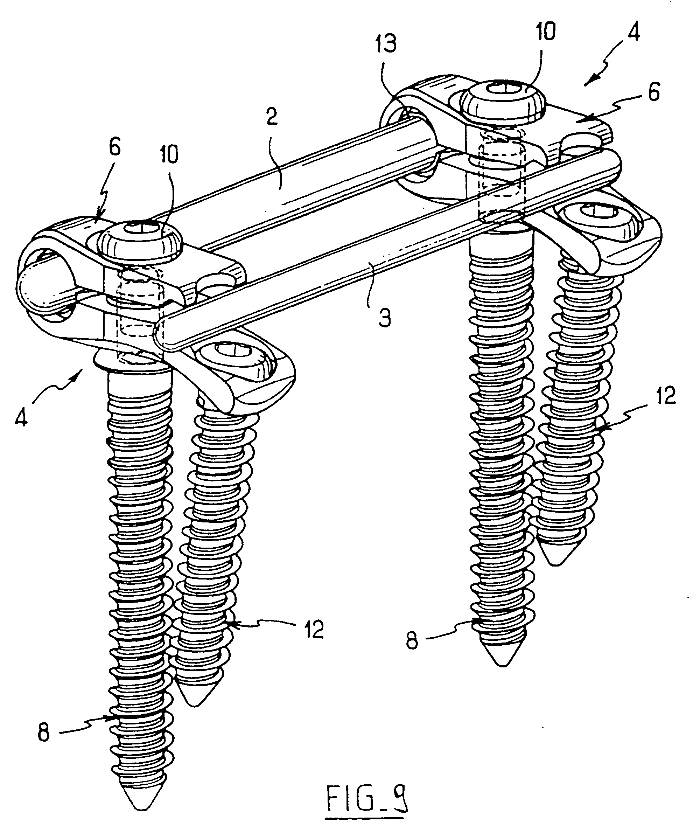

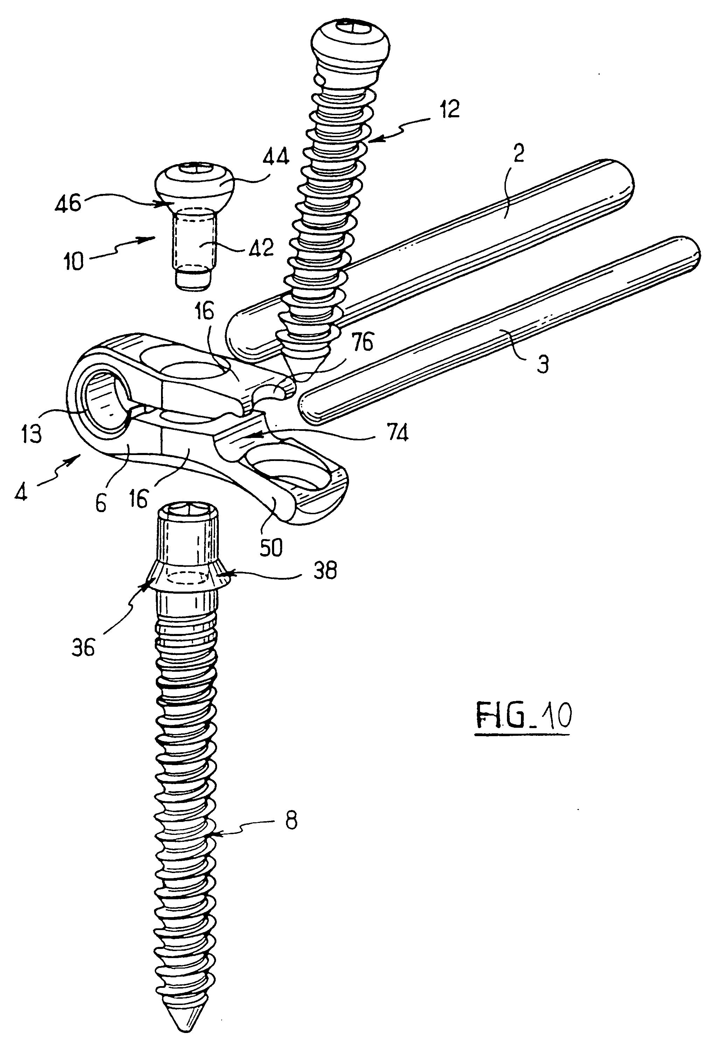

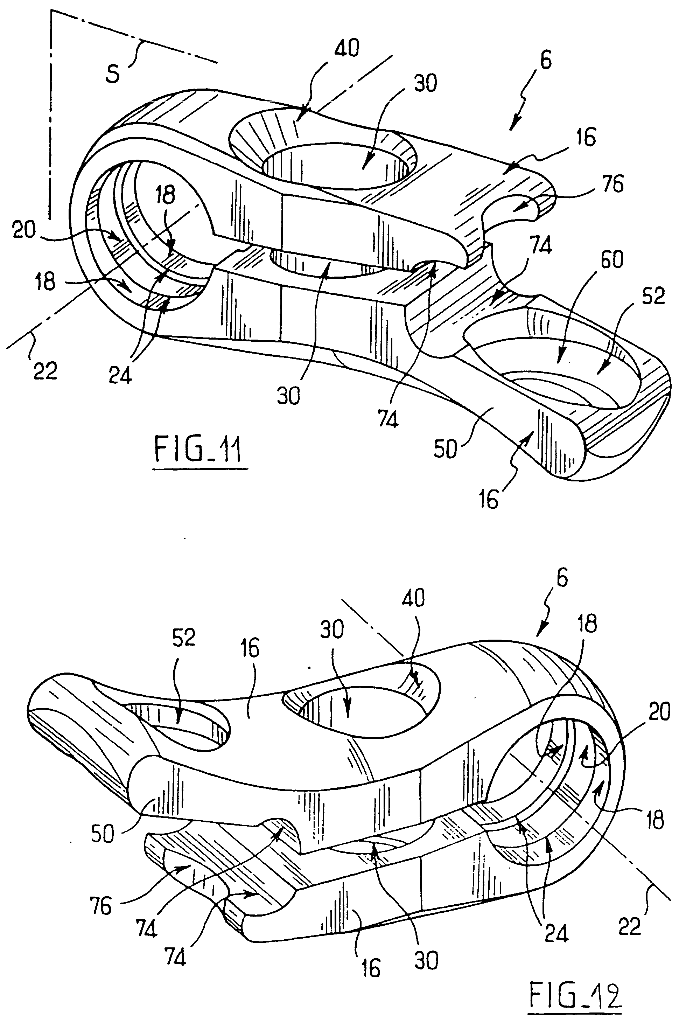

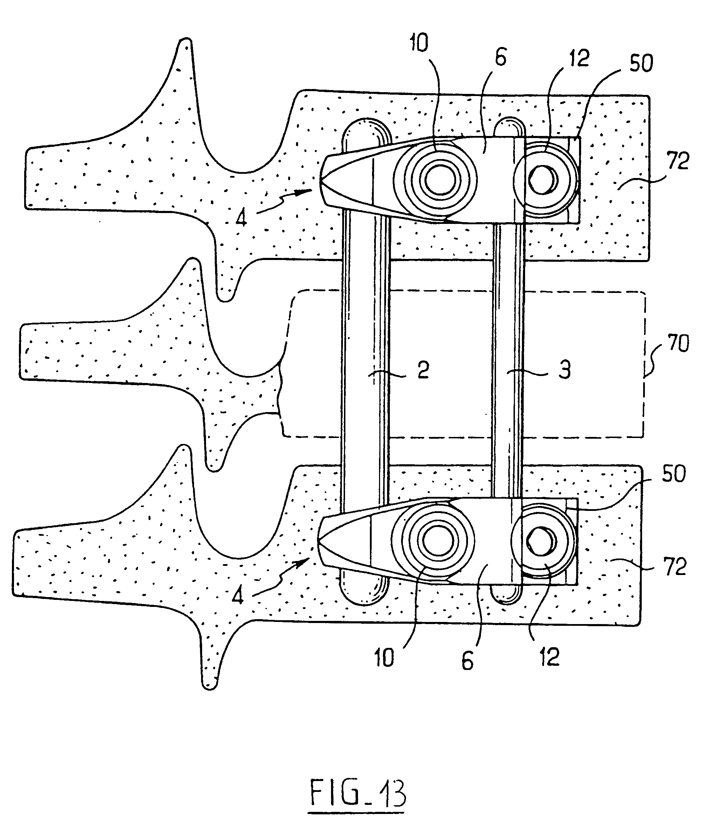

Dans le deuxième mode de réalisation illustré aux figures 9 à 13, le système est très proche de celui du premier mode. Il diffère cependant dans la présence d'une deuxième tige de liaison allongée 3 ou tige secondaire à section circulaire, et par l'adaptation du connecteur 6 à la réception de cette deuxième tige. La bague 13 est reçue sur la première tige ou tige principale 2.

Les deux tiges de liaison 2, 3 ont chacune une forme rectiligne profilée à profil ici circulaire. La tige secondaire 3 a une section, transversalement à son axe longitudinal, de diamètre inférieur à celui de la tige principale 2. La tige principale 2 aura par exemple un diamètre de 6 mm. Par exemple, le diamètre de la tige secondaire 3 sera compris entre 30% et 80% du diamètre de la tige principale 2. Ce faible diamètre permet au chirurgien de choisir la courbure de la tige secondaire 3 qui correspond à celle de l'étage du rachis instrumenté. En revanche, puisque les bagues 13 permettent une angulation relative entre les deux connecteurs 6, la tige principale 2 n'a pas besoin d'être cintrée. Elle peut donc avoir un diamètre important pour être très robuste.

Les branches 16 du connecteur présentent des empreintes cylindriques ou mors 74 respectives, ménagées dans les faces des branches en regard l'une de l'autre. Les empreintes 74 s'étendent en regard l'une de l'autre et ont des axes parallèles entre eux, et perpendiculaires au plan de symétrie S.

Sur la branche supérieure 16, l'empreinte 74 s'étend à une extrémité libre de la branche de sorte que l'orifice 30 est interposé entre les faces 18, 20 d'une part, et l'empreinte 74, d'autre part. Sur la branche inférieure 16, l'empreinte 74 s'étend entre les deux orifices 30 et 52, à la naissance du prolongement 50. Elle est contiguë à l'orifice 52 de sorte qu'elle mord sur son bord 60.

La tige secondaire 3 est destinée à être reçue dans l'empreinte 74 de la branche inférieure 16 dans une position angulaire unique par rapport au connecteur, perpendiculaire au plan de symétrie S. Lorsqu'on serre les deux branches 16 en direction l'une de l'autre, l'empreinte 74 de la branche supérieure vient en contact avec la tige secondaire 3 qui se trouve ainsi en contact surfacique avec chacune des deux empreintes qui réalisent un blocage à friction de la tige secondaire 3 par rapport au connecteur 6, lesquels sont ainsi rigidement fixés l'un à l'autre.

La tige secondaire 3 est placée dans l'empreinte 74 de la branche inférieure après que la vis secondaire 12 a été introduite dans l'orifice 52. La position de l'empreinte 74 de la branche inférieure fait en sorte que la tige secondaire 3 s'étend alors dans le trajet de la tête de la vis secondaire 12 pour son désengagement du connecteur et sa sortie de l'orifice 52. Par conséquent, une fois la tige secondaire 3 fixée au connecteur, la vis secondaire 12 ne peut plus être séparée du connecteur.

La branche supérieure 16 du connecteur présente à son extrémité libre une encoche 76 qui mord sur l'empreinte 74 à laquelle elle est contiguë et facilite la manoeuvre au moyen d'un outil de la vis secondaire 12 en dépit de l'encombrement de la branche supérieure.

La mise en place du système selon le deuxième mode est similaire à celle du système du premier mode. La mise en place des vis principale 8 et secondaire 12 est inchangée.

Après avoir instrumenté les deux vertèbres adjacentes 72, on positionne la tige principale 2 dans les bagues 13 des connecteurs 6 et on règle la position angulaire de chaque sous-ensemble 4 par rapport à cette tige 2. On introduit ensuite la tige secondaire 3 dans les empreintes 74 des connecteurs 6 après l'avoir préalablement cintrée manuellement pour obtenir la courbure requise à l'étage rachidien correspondant. En cas de défaut, cette tige 3 peut être ôtée pour corriger sa courbure puis replacée. La figure 9 montre le système avant serrage des branches. Le serrage final s'opère grâce à la vis de serrage 10 qui s'insère dans la vis principale 8 et comprime ainsi le connecteur 6 pour serrer ses deux branches 16 l'une vers l'autre. Durant ce serrage, l'effort de serrage est dirigé d'abord sur la tige principale 2 via la bague 13, jusqu'à ce que l'empreinte 74 de la branche supérieure vienne en contact avec la tige secondaire 3. Dès lors, l'effort de serrage est réparti sur les deux tiges 2, 3. Ainsi, la réaction au niveau du couple vis principale 8/vis de serrage 10 est sensiblement coaxiale à celles-ci.

Lorsque le système est en place, les connecteurs 6, au moins au nombre de deux, sont rigidement et simultanément fixés chacun aux mêmes tiges principale et tige secondaire.

On pourra indépendamment de la présence du prolongement 50 et de la deuxième vis 12, mettre en oeuvre les caractéristiques relatives à l'association de la première vis 8 avec la vis de serrage 10.

Bien que cela soit moins avantageux, la branche prolongée pourra être celle destinée à être la plus distante de la vertèbre.

On pourra mettre en oeuvre les caractéristiques relatives à la présence des deux vis vertébrales sur le connecteur indépendamment de celles relatives à la présence des tiges principale et secondaire, et réciproquement.

## Revendications

1. Système d'ostéosynthèse rachidienne, notamment de fixation antérieure, comprenant un élément de liaison allongé (2), une première vis vertébrale (8), et un connecteur (6) comportant deux branches (16) aptes à serrer entre elles l'élément de liaison (2), au moins une première des branches (16) étant apte à être engagée sur la première vis vertébrale (8), le système comprenant une deuxième vis vertébrale (12), la première branche (16) présentant un prolongement (50) apte à être engagé sur la deuxième vis (12), **caractérisé en ce que** le prolongement est disposé de sorte que les deux vis s'etendent d'un même côté de l'élément de liaison.

2. Système selon la revendication 1, **caractérisé en ce que** le prolongement (50) présente un évidement (52) de réception de la deuxième vis (12).

3. Système selon la revendication 2, **caractérisé en ce que** le prolongement (50) présente une empreinte sphérique (60) à un bord de l'évidement (52) destiné à être opposé à la vertèbre.

4. Système selon l'une quelconque des revendications 1 à 3, **caractérisé en ce qu'**une des branches (16) destinée à être opposée à la vertèbre présente un évidement (30) de réception de la première vis (8), et une empreinte sphérique (40) à un bord de l'évidement destiné à être opposé à la vertèbre.

5. Système selon l'une quelconque des revendications 1 à 4, **caractérisé en ce que** la branche prolongée (16) est apte à être cintrée manuellement, notamment au moyen d'un outil.

6. Système selon l'une quelconque des revendications 1 à 5, **caractérisé en ce que** la première vis (8) comporte une tête (32) et une collerette (36) distincte de la tête et apte à coopérer avec une des branches (16) destinée à être adjacente à la vertèbre, pour immobiliser le connecteur (6) en rotation par rapport à la première vis (8).

7. Système selon la revendication 6, **caractérisé en ce que** la collerette (36) présente une face (38), notamment conique, apte à immobiliser le connecteur (6) par friction.

8. Système selon l'une quelconque des revendications 1 à 7, **caractérisé en ce que** la première vis (8) présente un orifice fileté (39), le système comprenant une vis de serrage (10) apte à constituer une liaison vis-écrou avec cet orifice et à prendre appui sur une des branches (16) destinée à être opposée à la vertèbre pour serrer les branches (16).

9. Système selon l'une quelconque des revendications 1 à 8, **caractérisé en ce qu'**il comprend une bague (13) apte à être enfilée sur l'élément de liaison (2) et reçue entre les branches (16), le connecteur (6) et la bague (13) étant conformés pour permettre un réglage de l'orientation de l'élément de liaison (2) dans deux plans perpendiculaires entre eux, avant le serrage des branches (16).

10. Système selon l'une quelconque des revendications 1 à 9, **caractérisé en ce que** les deux branches (16) font partie d'une même pièce (6) déformable élastiquement pour rapprocher les branches l'une de l'autre.

11. Système selon l'une quelconque des revendications 1 à 10, **caractérisé en ce que** le connecteur (6) est apte à être fixé à la vis vertébrale (8) et au premier élément de liaison (2) en choisissant une position angulaire de l'élément de liaison par rapport au connecteur.

12. Système selon l'une quelconque des revendications 1 à 11, **caractérisé en ce qu'**il comprend un deuxième élément de liaison allongé (3), le connecteur (6) étant apte à être fixé simultanément aux deux éléments de liaison (2, 3).

13. Système selon la revendication 12, **caractérisé en ce qu'**il est agencé de sorte que le deuxième élément de liaison (3) ne peut être fixé au connecteur (6) que dans une seule position angulaire par rapport au connecteur.

14. Système selon la revendication 12 ou 13, **caractérisé en ce que** le deuxième élément de liaison (3) présente une résistance au cintrage plus faible que le premier élément de liaison (2).

15. Système selon l'une quelconque des revendications 12 à 14, **caractérisé en ce que** les branches (16) sont aptes à serrer simultanément les deux éléments de liaison (2, 3).

16. Système selon l'une quelconque des revendications 12 à 15, **caractérisé en ce qu'**il est agencé de sorte que le deuxième élément de liaison (3), lorsqu'il est fixé au connecteur (6), s'étend dans une trajectoire de la deuxième vis vertébrale (12) pour son désengagement du connecteur (6).

17. Système selon l'une quelconque des revendications 12 à 16, **caractérisé en ce qu'**il comprend un deuxième connecteur, les deux connecteurs (6) étant aptes à être fixés simultanément chacun aux deux éléments de liaison (2, 3).

## Patentansprüche

1. Wirbelsäulen-Osteosynthesesystem besonders für die vordere Fixierung, das ein längliches Verbindungselement (2), eine erste Wirbelschraube (8) und ein Anschlußstück (6) umfaßt, das zwei Schenkel (16) umfaßt, die geeignet sind, das Verbindungselement (2) zwischeneinander einzuklemmen, wobei mindestens ein erster der Schenkel (16) geeignet ist, auf der ersten Wirbelschraube (8) in Eingriff gebracht zu werden, wobei das System eine zweite Wirbelschraube (12) umfaßt, und wobei der erste Schenkel (16) eine Verlängerung (50) umfaßt, die geeignet ist, auf der zweiten Schraube (12) in Eingriff gebracht zu werden, **dadurch gekennzeichnet, daß** die Verlängerung derart angeordnet ist, daß sich die beiden Schrauben von ein und derselben Seite des Verbindungselementes aus erstrecken.

2. System nach Anspruch 1, **dadurch gekennzeichnet, daß** die Verlängerung (50) eine Ausnehmung (52) zur Aufnahme der zweiten Schraube (12) aufweist.

3. System nach Anspruch 2, **dadurch gekennzeichnet, daß** die Verlängerung (50) eine kugelige Vertiefung (60) an einem Rand der Ausnehmung (52) aufweist, der dazu geeignet ist, vom Wirbel wegzuweisen.

4. System nach einem der Ansprüche 1 bis 3, **dadurch gekennzeichnet, daß** einer der Schenkel (16), der dazu bestimmt ist, vom Wirbel wegzuweisen, eine Ausnehmung (30) zur Aufnahme der ersten Schraube (8) und eine kugelige Vertiefung (40) an einem Rand der Ausnehmung aufweist, der dazu bestimmt ist, vom Wirbel wegzuweisen.

5. System nach einem der Ansprüche 1 bis 4, **dadurch gekennzeichnet, daß** der längliche Schenkel (16) geeignet ist, von Hand, besonders mittels eines Werkzeugs, gebogen zu werden.

6. System nach einem der Ansprüche 1 bis 5, **dadurch gekennzeichnet, daß** die erste Schraube (8) einen Kopf (32) und einen Kragen (36) umfaßt, der vom Kopf unterschieden ist und geeignet ist, mit einem der Schenkel (16) zusammenzuwirken, um das Anschlußstück (6) bezüglich der ersten Schraube (8) drehunbeweglich zu machen.

7. System nach Anspruch 6, **dadurch gekennzeichnet, daß** der Kragen (36) eine insbesondere konische Fläche (38) aufweist, die geeignet ist, das Anschlußstück (6) durch Reibung unbeweglich zu machen.

8. System nach einem der Ansprüche 1 bis 7, **dadurch gekennzeichnet, daß** die erste Schraube (8) eine mit Gewinde versehene Öffnung (39) aufweist, und daß das System eine Klemmschraube (10) umfaßt, die geeignet ist, eine Schrauben-Mutter-Verbindung mit dieser Öffnung zu bilden und gegen einen der Schenkel (16) anzuliegen, der dazu bestimmt ist, vom Wirbel wegzuweisen, um die Schenkel (16) festzuklemmen.

9. System nach einem der Ansprüche 1 bis 8, **dadurch gekennzeichnet, daß** es einen Ring (13) umfaßt, der geeignet ist, auf dem Verbindungselement (2) aufgezogen zu werden und zwischen den Schenkeln (16) aufgenommen zu werden, und daß das Verbindungsstück (6) und der Ring (13) dazu ausgebildet sind, eine Regulierung der Richtung des Verbindungselementes (2) in zwei zueinander senkrechten Ebenen vor dem Festklemmen der Schenkel (16) zu gestatten.

10. System nach einem der Ansprüche 1 bis 9, **dadurch gekennzeichnet, daß** die beiden Schenkel (16) Teil ein und desselben Stückes (6) bilden, das elastisch verformbar ist, um die Schenkel aneinander anzunähern.

11. System nach einem der Ansprüche 1 bis 10, **dadurch gekennzeichnet, daß** das Verbindungsstück (6) dazu geeignet ist, an der Wirbelschraube (8) und am ersten Verbindungselement (2) befestigt zu werden, indem man eine Winkellage des Verbindungselements bezüglich des Verbindungsstückes wählt.

12. System nach einem der Ansprüche 1 bis 11, **dadurch gekennzeichnet, daß** es ein zweites, längliches Verbindungselement (3) umfaßt, wobei das Anschlußstück (6) geeignet ist, gleichzeitig an den beiden Verbindungselementen (2, 3) befestigt zu werden.

13. System nach Anspruch 12, **dadurch gekennzeichnet, daß** es derart eingerichtet ist, daß das zweite Verbindungselement (3) nur in einer einzigen Winkellage in Bezug auf das Verbindungsstück (6) an diesem befestigt werden kann.

14. System nach Anspruch 12 oder 13, **dadurch gekennzeichnet, daß** das zweite Verbindungselement (3) einen schwächeren Biegewiderstand aufweist als das erste Verbindungselement (2).

15. System nach einem der Ansprüche 12 bis 14, **dadurch gekennzeichnet, daß** die beiden Schenkel (16) dazu geeignet sind, gleichzeitig die zwei Verbindungselemente (2, 3) festzuklemmen.

16. System nach einem der Ansprüche 12 bis 15, **dadurch gekennzeichnet, daß** es derart angeordnet ist, daß das zweite Verbindungselement (3) dann, wenn es am Anschlußstück (6) befestigt ist, sich für ein Lösen des Anschlußstücks (6) in einer Bewegungsbahn der zweiten Wirbelschraube (12) erstreckt.

17. System nach einem der Ansprüche 12 bis 16, **dadurch gekennzeichnet, daß** es ein zweites Anschlußstück umfaßt, wobei die beiden Anschlußstücke (6) geeignet sind, gleichzeitig jeweils an den beiden Verbindungselementen (2, 3) befestigt zu werden.

## Claims

1. Spinal osteosynthesis system, in particular for anterior fixation, comprising an elongate connection element (2), a first vertebral screw (8), and a connector (6) including two branches (16) which are able to clamp the connection element (2) between them, at least a first of the branches (16) being able to be engaged on the first vertebral screw (8), the system comprising a second vertebral screw (12), the first branch (16) having an extension (50) which can be engaged on the second screw (12), **characterized in that** the extension is disposed in such a way that both screws extend on the same side of the connection element.

2. System according to Claim 1, **characterized in that** the extension (50) has an opening (52) for receiving the second screw (12).

3. System according to Claim 2, **characterized in that** the extension (50) has a spherical recess (60) at one edge of the opening (52) intended to be remote from the vertebra.

4. System according to any one of Claims 1 to 3, **characterized in that** one of the branches (16) which is intended to be remote from the vertebra has an opening (30) for receiving the first screw (8), and a spherical recess (40) at one edge of the opening intended to be remote from the vertebra.

5. System according to any one of Claims 1 to 4, **characterized in that** the extended branch (16) can be bent manually, in particular using a tool.

6. System according to any one of Claims 1 to 5, **characterized in that** the first screw (8) includes a head (32) and a flange (36) distinct from the head and able to cooperate with one of the branches (16) which is intended to be adjacent to the vertebra, in order to immobilize the connector (6) in terms of rotation relative to the first screw (8).

7. System according to Claim 6, **characterized in that** the flange (36) has a face (38), which is in particular a conical face, able to immobilize the connector (6) by friction.

8. System according to any one of Claims 1 to 7, **characterized in that** the first screw (8) has a threaded orifice (39), the system comprising a clamping screw (10) which can constitute a screw-nut connection with this orifice and is able to bear on one of the branches (16) which is intended to be remote from the vertebra in order to clamp the branches (16).

9. System according to any one of Claims 1 to 8, **characterized in that** it comprises a ring (13) which can be engaged on the connection element (2) and received between the branches (16), the connector (6) and the ring (13) being designed to permit control of the orientation of the connection element (2) in two mutually perpendicular planes before the branches (16) are clamped.

10. System according to any one of Claims 1 to 9, **characterized in that** the two branches (16) form part of a single component (6) which is elastically deformable in order for the branches to be closed towards each other.

11. System according to any one of Claims 1 to 10, **characterized in that** the connector (6) can be fixed to the vertebral screw (8) and to the first connection element (2) by choosing an angular position of the connection element relative to the connector.

12. System according to any one of Claims 1 to 11, **characterized in that** it comprises a second elongate connection element (3), the connector (6) being able to be fixed simultaneously to the two connection elements (2, 3).

13. System according to Claim 12, **characterized in that** it is designed in such a way that the second connection element (3) can be fixed to the connector (6) only in a single angular position relative to the connector.

14. System according to Claim 12 or 13, **characterized in that** the second connection element (3) has less resistance to bending than the first connection element (2).

15. System according to any one of Claims 12 to 14, **characterized in that** the branches (16) can simultaneously clamp the two connection elements (2, 3).

16. System according to any one of Claims 12 to 15, **characterized in that** it is designed in such a way that the second connection element (3), when fixed to the connector (6), extends in a trajectory of the second vertebral screw (12) for its disengagement from the connector (6).

17. System according to any one of Claims 12 to 16, **characterized in that** it comprises a second connector, the two connectors (6) each being able to be fixed simultaneously to the two connection elements (2, 3).
